# EUROPEAN PATENT APPLICATION

(11) **EP 2 781 207 A1**
(43) Date of publication of application: **24.09.2014**
(21) Application number: 13160424.1
(22) Date of filing: 21.03.2013
(51) Int. Cl.: A61F 9/007, A61F 9/00

(54) **Apparatus for application on an eye**

(71) Applicant: Simader, Christian, 1220 Vienna (AT); Gerendas, Bianca, 1190 Wien (AT)
(72) Inventor: Simader, Christian, 1220 Vienna (AT); Gerendas, Bianca, 1190 Wien (AT)
(74) Representative: Sonn & Partner Patentanwälte

(57) **Abstract**

An apparatus (1) for application on an eye (5), comprising an eye bearing element (2) with a lower surface (3) for bearing on the eye (5), the eye bearing element (2) comprising an insertion area (13) for passing an ophthalmic instrument (7), in particular an injection needle (7') or a surgical instrument (7"), therethrough, wherein the eye bearing element (2), at least in the insertion area (13), is made from a resilient material arranged such that an insertion aperture (14) formed in the insertion area (13) upon insertion of the ophthalmic instrument (7) is at least substantially closed when the ophthalmic instrument (7) is retracted from the eye bearing element (2).

## Description

The present invention relates to an apparatus for application on an eye, comprising an eye bearing element with a lower surface for bearing on the eye, the eye bearing element comprising an insertion area for passing an ophthalmic instrument, in particular an injection needle or a surgical instrument, therethrough.

In the art, a number of devices have been developed to deliver drugs into the eyes of humans or animals. Intraocular injection is commonly used in ophthalmology for delivering high concentrations of therapeutics to the eye. As an example, anti-VEGF (vascular endothelial growth factor) may be administered for treating different kinds of diseases affecting the eye, age-related macular degeneration being one such disease.

Document WO 2008/084063 A1 discloses an apparatus comprising a plate for being brought into contact with an eye and a support for receiving a syringe. The plate has an eye bearing surface having a curved shape for matingly bearing on the outer surface of the eye. Furthermore, the plate has a cut-out having an edge having a substantially circular shape which corresponds to a shape of a limbus so that the edge can be superimposed on the limbus. The cut-out serves as a reference for precisely positioning the apparatus with respect to the eye in order to perform an intra-ocular injection. The plate is provided with an aperture for allowing a needle to pass through the plate.

This prior art also discusses the problem that perforation of the tissues can cause leakage of the injected composition outside the eye through the orifice created by the injection needle, such that the user is prevented from controlling the amount of active compound that has been actually introduced into the eye. As a solution to this problem, the known apparatus also comprises a resilient member for displacing a superficial layer of the eye over an underlying layer of the eye. The resilient member comprises a flexible leg projecting outwardly from the plate from the side of the bearing surface and a plurality of teeth arranged at the free end of the flexible leg. When the apparatus is removed after injection of the substance, the resilient member is moved away from the eye. As a consequence, the superficial layer slides over the underlying layer, back to its initial position. The orifice created in the superficial layer by the needle is shifted relative to the orifice created in the underlying layer. As the orifice is obstructed by the superficial layer, the composition which has been injected into the vitreous body of the eye is prevented from leaking out of the eye. Moreover, this also avoids penetration of germs into the eye.

However, it has been demonstrated that the reflux of liquid frequently may occur despite the repositioning of the superficial layer after injecting the drugs. Furthermore, the degree of leakage remains hardly predictable. Also, even if leakage from the eye was prevented, the drugs could accumulate between the superficial and the underlying layer of the eye. However, drugs misplaced in this way may not yield the desired effect.

Furthermore, it has been attempted to reduce the leakage of eye liquid by obliquely piercing the sclera of the eye in order to increase the resistance against a reflux of liquid through this elongated incision. However, as a drawback this technique requires high skill of the operator and fails to reliably prevent leakage.

On the other hand, the prior art comprises a great variety of surgical instruments used during surgery on the eye. Here too, the reflux of eye liquid poses a serious problem.

Document EP 1 858 459 discloses a surgical ophthalmic device for sealing an opening in the eye created during surgery. This device comprises an annular sealing element with a passageway for introducing a surgical instrument and a sealing region which is placed on the eye tissue surrounding the opening with a sealing surface. The sealing element is elastically deformable at least in the sealing region. However, in this device the sealing surface is spaced apart from the opening, such that eye liquid is allowed to leak through the space between the opening and the surgical instrument. In order to maintain a constant eye pressure, an irrigation liquid is constantly introduced to the eye. Thus, this technique could not be used for administering drugs into the eye while reliably preventing the leakage of liquid from the opening.

This document also discusses another prior art technique, where the surgical instrument, an ultrasonic needle, is surrounded by a sealing sleeve. However, this technique has the disadvantage that a comparatively large opening has to be created in the eye to accommodate the surgical instrument together with the sealing sleeve. As a further drawback, in this case it would not be possible to use standard injection needles. Furthermore, this prior art does not address the problem of reflux of eye liquid after retraction of the surgical instrument.

It is thus an object of the invention to provide for an improved ophthalmic device for the intraocular administration of substances, wherein the leakage of liquids through the orifice created by the injection needle can be reliably prevented, such that the amount of substance remaining in the eye may be precisely predicted.

According to an aspect of the invention, the eye bearing element, at least in the insertion area, is made from a resilient material arranged such that an insertion aperture formed in the insertion area upon insertion of the ophthalmic instrument is at least substantially closed when the ophthalmic instrument is retracted from the eye bearing element.

In a preferred embodiment of the invention, an intraocular injection procedure is performed, which generally comprises the steps of placing the eye bearing element on the superficial layer of the eye, introducing the ophthalmic instrument, in this case an injection needle, through the insertion area of the eye bearing element, creating an opening in the eye, and injecting a composition, e.g. anti-VEGF agents, antibiotics, cortisone, rtPA, air or other gases, via the injection needle. In another preferred embodiment, the apparatus is used with an elongate surgical instrument, e.g. a vitrectome.

After retraction of the ophthalmic instrument, the elasticity of the resilient material causes the insertion opening to close, which essentially prevents the leakage of the injected substance from the eye. As a result, the amount of the substance remaining in the eye, at the predetermined position, is known with highest possible precision.

In a preferred embodiment, the eye bearing element comprises a continuous insertion area arranged for producing the insertion aperture by means of piercing the insertion element therethrough. In an unused state, the continuous insertion area of the eye bearing element is free from an insertion aperture, which is only created when the ophthalmic instrument is pierced through the eye bearing element. The formation of the insertion aperture in the eye bearing element creates a restoring force in the resilient material around the insertion aperture, whereby the eye bearing element essentially returns to its originally state, when the ophthalmic instrument is withdrawn from the eye. Here, the elasticity of the resilient material is such that the insertion aperture created by the ophthalmic instrument is substantially closed when the ophthalmic instrument is retracted from the eye. Thus, the leakage of liquids from the eye is at least largely reduced or entirely prevented.

In an alternative preferred embodiment, the eye bearing element comprises a flap transferrable between a closed position, wherein the insertion aperture is closed, and an open position, wherein the insertion aperture is open, wherein the flap is biased towards the closed position. Thus, the insertion aperture is substantially closed when the ophthalmic instrument is retracted from the eye and the eye bearing element. This embodiment may be used in connection with a surgical instrument having a flat lower end. However, also an injection needle with a pointed tip may be used. For this purpose, it is preferable that the injection needle is provided with an attachment fixable or fixed to the lower end thereof. Preferably, the attachment or prolongation comprises a connecting portion for detachably connecting the attachment to the lower end of the injection needle. In particular, the connecting portion may be snapped on the injection needle. Furthermore, the attachment may comprise a middle portion which, in the attached state, comes into abutment with the lower end of the injection needle. The middle portion may protect the pointed tip of the injection needle and may also serve as a stop element when mounting the attachment on the injection needle. Preferably, the attachment further comprises a transition portion, which connects the middle portion with an insertion portion at the free end of the attachment. The free end of the insertion portion may be comparatively flat in order to avoid damaging the eye bearing element upon insertion of the attachment. It is particularly advantageous, if the insertion portion is flattened in cross-section in order to facilitate opening of the flap. Furthermore, the attachment preferably comprises a central channel for guiding the substance from the injection needle into the eye.

In an alternative embodiment, the insertion area of the eye bearing element comprises a permanent aperture arranged for sealingly accommodating the ophthalmic instrument. The resilient material around the permanent aperture of the eye bearing element forms a sealing surface, which may sealingly abut against the ophthalmic instrument. In operation, the resilient material around the permanent aperture is elastically widened to sealingly bear on the lateral surface of the ophthalmic instrument. Furthermore, the permanent aperture is dimensioned such that the leakage of liquid is prevented when the ophthalmic instrument is withdrawn from the eye bearing element.

In this case, it is preferred that a diameter of the permanent aperture of the injection area is smaller than a standardized diameter of an injection needle, in particular less than 0.36 mm. The standardized diameters of injection needles typically range between 27 gauge (0.36 mm) and 30 gauge (0.255 mm). Thus, in operation, the resilient material around the, circular-shaped, permanent aperture is pushed apart by the ophthalmic instrument, such that the sealing surface of the eye bearing element sealingly abuts against the surface of the ophthalmic instrument. After retraction of the ophthalmic instrument, the permanent aperture is elastically relaxed to its original size, which is dimensioned to essentially prevent leaking therefrom.

In view of sealing the orifice formed in the eye upon retraction of the ophthalmic instrument, it is preferred if the resilient material of the eye bearing element comprises a natural or synthetic rubber material. However, any resilient material with a sufficient elasticity may be used, e.g. an elastomer.

In a further preferred embodiment, the eye bearing element is made in one piece from the resilient material. As an advantage, the integral design of the eye bearing element allows for a cost-efficient production. Furthermore, the resilient material of the eye bearing element outside the insertion area is advantageously arranged for sealing the lower surface of the eye bearing element against the superficial layer of the eye.

In a further preferred embodiment, the lower surface of the eye bearing element is curved for matingly bearing on the eye, the radius of curvature of the eye bearing element preferably being less than the radius of curvature of a human eye. In this case, the central insertion region of the eye bearing element is tightly pressed against the surface of the eye. Here, the curvature of the human eye, which varies marginally among individuals, outside the cornea is taken. As an alternative, the eye bearing element may be essentially planar. In this case, the resilient material of the eye bearing element is deformable to matingly bear on the superficial layer of the eye, which itself is deformable to some degree.

In a further preferred embodiment, the eye bearing element is connected to a guiding device for guiding the ophthalmic instrument between a retracted position, wherein the tip of the ophthalmic instrument is retracted inside the guiding device, and an inserted position, wherein the tip of the ophthalmic instrument protrudes outside the guiding device. Preferably, the guiding device comprises a stop element which restricts protrusion of the ophthalmic instrument outside the guiding device. In this case, the depth and angle of insertion of the ophthalmic instrument into the eye is predetermined by the arrangement of the stop element.

In a further preferred embodiment, the guiding device comprises pressurizing means for passing a pressurized fluid to the injection area of the eye bearing element. In this case, the pressurizing means are arranged for applying pressure in the area of the orifice created by the ophthalmic instrument. Thus, the pressurizing means assist in preventing leakage of liquids from the orifice. This embodiment is particularly advantageous in case the eye bearing element comprises a permanent aperture, as described above. The pressurizing means are preferably active at least during and right after administration of the substance into the eye. However, it is particularly advantageous that the pressurizing means remain active for a predetermined duration after the ophthalmic instrument has been retracted from the eye.

For applying a pressure to the injection zone it is preferred that the pressurizing means comprise a pressure chamber inside the guiding device, the pressure chamber having an inlet for connecting a source of pressurized fluid thereto and an outlet for passing the pressurized fluid to the injection area of the eye bearing element. The source of pressurized fluid may for example supply pressurized air or water.

In order to maintain the pressure inside the pressure chamber it is preferred that the guiding device comprises a passageway for passing the ophthalmic instrument therethrough, the passageway extending to the pressure chamber, wherein the guiding device further comprises a shut-off member for shutting off the passageway when the ophthalmic instrument is arranged in the retracted position. It is also preferred that the shut-off member is coupled to a spring member such that the shut-off member is forced into the shut-off position, when the ophthalmic instrument is retracted beyond the shut-off member. In this case, the passageway is automatically shut-off when the ophthalmic instrument is removed from the apparatus.

In a further preferred embodiment, the eye bearing element comprises at least one paracentral opening outside the insertion area.

In view of improving the contact between the eye bearing element and the superficial layer of the eye (conjunctiva or sclera), it is preferred that the at least one paracentral opening of the eye bearing element is in fluid communication with at least one vacuum chamber inside the guiding device, the vacuum chamber having an intake for connecting a vacuum source thereto.

In an alternative embodiment, the apparatus comprises a spring element arranged for application of a predetermined pressure onto the paracentral region of eye bearing element when the injection needle is introduced into the eye.

According to preferable constructions of the apparatus, the eye bearing element is made in one piece with the guiding device or the eye bearing element is detachably mounted on the guiding device. In the former embodiment, a cheap, easy-to-manufacture apparatus is obtained, which is particularly intended for one way use, however, may also be reused. On the other hand, the latter embodiment allows for the replacement of a used eye bearing element in preparation of at least a second insertion procedure.

In the following, the invention will be explained by way of preferred embodiments illustrated in the drawings, yet without being restricted thereto. In the drawings:
Fig. 1 shows an apparatus for application on a human eye according to a first embodiment, comprising an eye bearing element made from a resilient material, wherein an initially continuous insertion area (Fig. 1a) of the eye bearing element is pierced by introduction of an ophthalmic instrument (Fig. 1b);
Fig. 2a to Fig. 2f show an apparatus according to a second embodiment in different stages of an injection procedure;
Fig. 3a, 3b show an apparatus according to a third preferred embodiment, wherein the eye bearing element comprises a flap transferrable between a closed position (Fig. 3a) and an open position (Fig. 3b) by means of the ophthalmic instrument;
Fig. 3c shows a variation of the embodiment according to Fig. 3a, 3b, wherein the ophthalmic instrument at the lower end comprises an attachment for moving the flap between the closed and the open position; and
Fig. 4 shows an apparatus according to a fourth embodiment, wherein the eye bearing element comprises a permanent insertion aperture for the ophthalmic instrument.

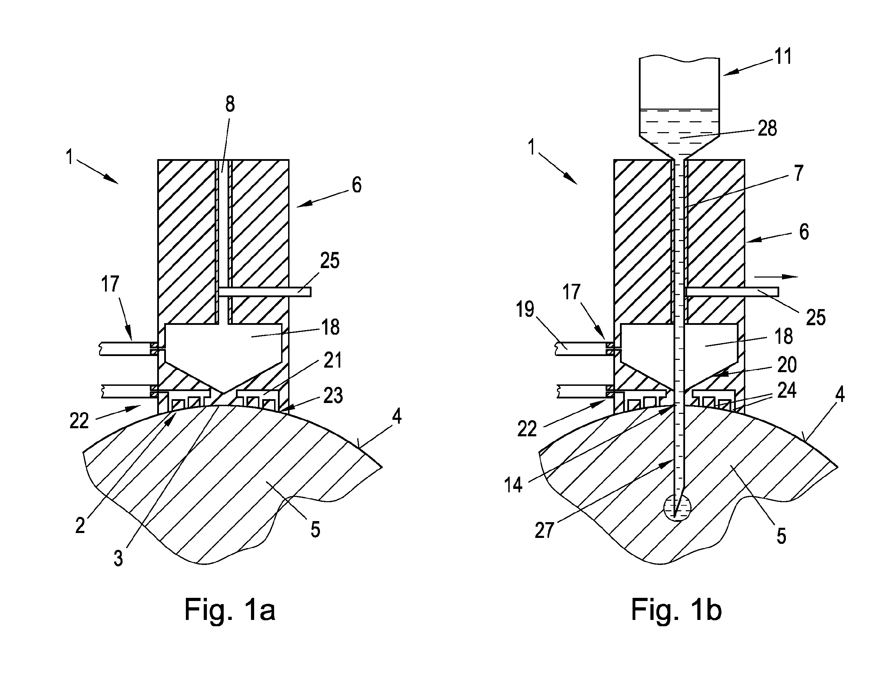

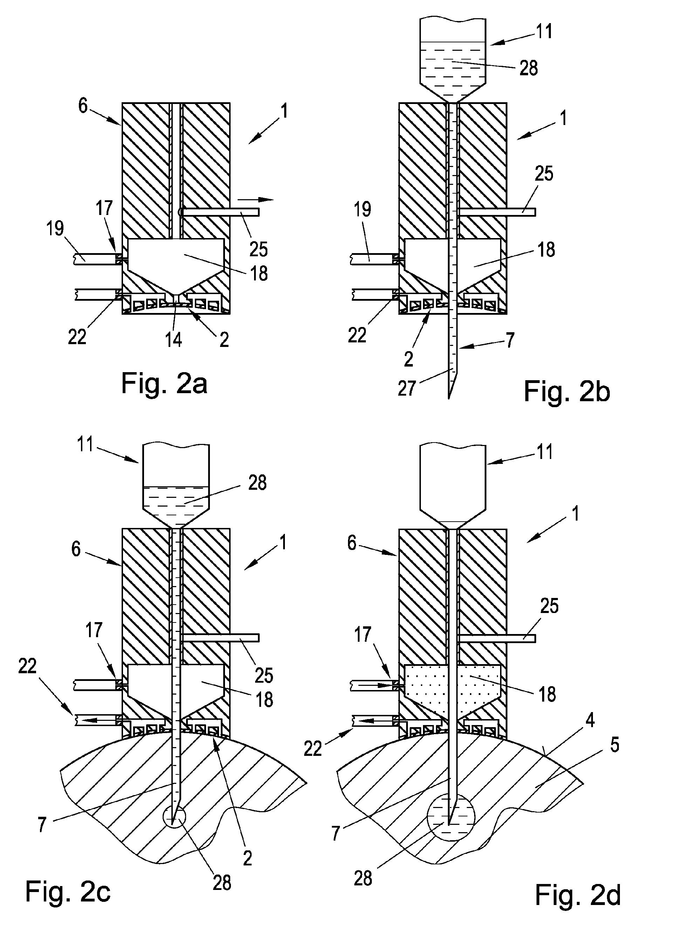

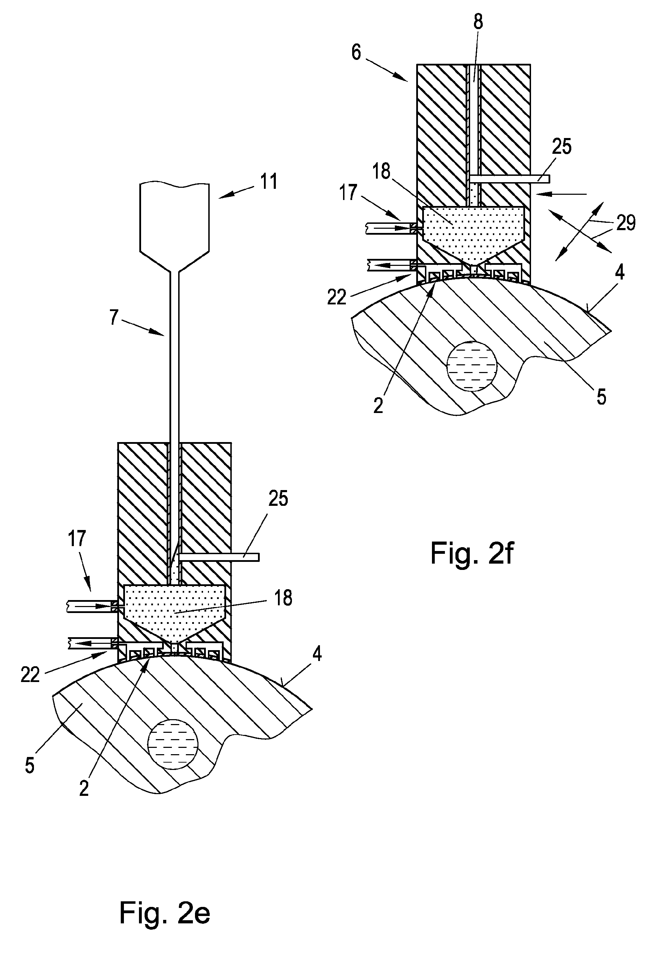

Fig. 1 illustrates an apparatus 1 for intraocular, in particular intravitreal, injection according to a first preferred embodiment of the invention. The ophthalmic apparatus 1 comprises an eye bearing element 2 with a lower surface 3 for bearing on the eye. The lower surface 3 of the eye bearing element 2 is concavely curved for matingly bearing on the superficial layer 4 of an eye 5 (cf. Fig. 2). Preferably, the curvature of the eye bearing element 2 outside the cornea is less than the curvature of the eye 5 such that the pressure applied at the site of injection is higher than the pressure difference between the pressure inside the eye 5 and the pressure outside the eye 5, the latter being atmospheric pressure. The lower surface 3 may deviate from a rotationally symmetric shape. The ophthalmic apparatus 1 further comprises a guiding device 6 for guiding an ophthalmic instrument 7, here an injection needle 7' with a pointed tip for administration of a drug into the eye 5, during an injection procedure (cf. Fig. 2). In one embodiment, the guiding device 6 comprises a generally cylindrical shape with an essentially circular cross-section. Alternatively, the guiding device 6 may be oval, preferably elliptical, in cross-section. Moreover, the guiding device 6 comprises an internal passageway 8 for passing the injection needle 7' therethrough. The passageway 8 terminates at an opening 9 for insertion of the ophthalmic instrument 7. The insertion opening 9 is arranged at a first front end 10 of the guiding device 6, which serves as a stop element or bearing surface for a syringe 11 carrying the injection needle 7' (cf. Fig. 2). On the other hand, the eye bearing element 2 is attached to a second front end 12 of the guiding device 6, which is shaped corresponding to the eye bearing element 2. The eye bearing element 2 comprises an insertion area 13 for passing the ophthalmic instrument 7 therethrough.

Moreover, the eye bearing element 2, at least in the insertion area 13, is made from a resilient material arranged such that an insertion aperture 14 formed in the insertion area 13 is at least substantially closed when the ophthalmic instrument 7 is retracted from the eye bearing element 2. In this case, the leakage of eye liquids may be prevented or at least largely reduced. As can be seen from the drawings, the insertion area 13 is preferably formed at the central region of the eye bearing element 2.

In all embodiments, the insertion aperture 14 is present when the ophthalmic instrument 7 is passed through the insertion area 13. However, the embodiments differ in the formation of the insertion aperture 14.

According to the Fig. 1a, 1b, the eye bearing element 2 comprises a continuous injection area 13 made from the resilient material. For the purpose of this disclosure, the term "continuous" refers to the lack of a permanent aperture in the injection area 13. Fig. 1a shows the eye bearing element 2 in an unused state, wherein the continuous insertion area 13 is formed by resilient material. In operation, the injection needle 7' is pierced through the insertion area 13 of the eye bearing element 2 for producing the insertion aperture 14 (cf. Fig. 1b). In this case, the resilient material around the injection aperture 14 created by the insertion needle 7' sealingly abuts against the outer surface of the injection needle 7' pierced through the insertion area 13. In the inserted state of the ophthalmic instrument 7, the insertion aperture 14 is sized according to the cross-section of the ophthalmic instrument 7. When the ophthalmic instrument 7 is withdrawn from the eye 5 and the eye bearing element 2, the aperture 14 formed by means of the ophthalmic instrument 7 is closed due to the elasticity of the resilient material. Thus, the leakage of liquid from the eye 5 through the insertion aperture 14 is prevented.

According to Fig. 1, the eye bearing element 2 is made in one piece with the guiding device 6. However, the eye bearing element 2 may also be separate from the guiding device 6 to allow for a replacement of the eye bearing element 2 after continued use, as in the embodiment of Fig. 2.

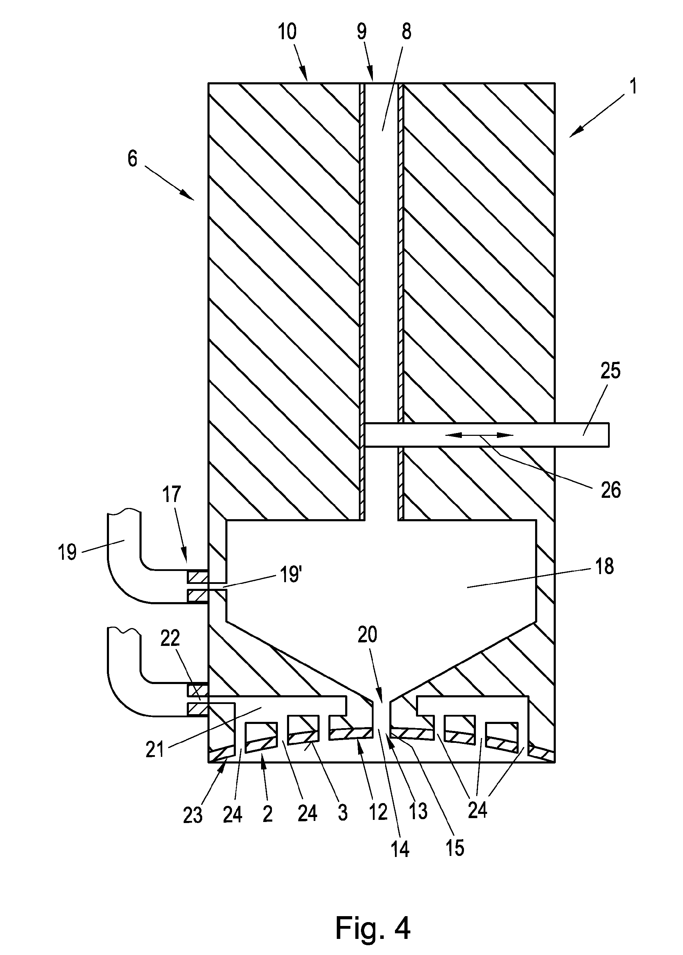

Moreover, the guiding device 6 comprises pressurizing means 17 for passing a pressurized fluid to the injection area 13 of the eye bearing element 2. Thus, a fluid pressure can be applied to the injection area 13, which aids in preventing the leakage of liquids from the eye 5 after retraction of the injection needle 7'. For this purpose, the pressurizing means 17 are arranged for further biasing the resilient material of the eye bearing element 2 against the superficial layer 4 of the eye 5 (cf. Fig. 2). Thus, the pressurizing means 17 may pressurize the outer surface of the eye 5 in the area of the orifice created by the injection needle 7'. In this way, the pressurizing means 17 further impede the leakage of eye liquids and drug outside the orifice. The pressurizing means 17 comprise a pressure chamber 18 inside the guiding device 6. The pressure chamber 18 comprises an inlet 19' arranged on the lateral surface of the guiding device 6. The inlet 19' receives pressurized fluid from a conduit 19, which is connected to a source of pressurized fluid, e.g. a balanced salt solution or air (cf. Fig. 4). The pressure chamber 18 further comprises a funnel-shaped outlet 20, which extends to the permanent opening 14 of the eye bearing element 2. Thus, the pressurizing means 17 convey a current of pressurized fluid via the pressure chamber 18 to the injection area 13 of the eye bearing element 2 during and after insertion of the injection needle 7'.

Moreover, the guiding device 6 comprises a vacuum chamber 21 for applying a negative pressure to the paracentral region 23 of the eye bearing element 2 outside the injection area 13. The paracentral region of the eye bearing element 2 comprises paracentral, for example circular openings 24, which are in fluid communication with the vacuum chamber 21 of the guiding device 6. The vacuum chamber 21 comprises an intake 22 for connecting a vacuum source (not shown) thereto. In operation, the vacuum source creates a negative pressure inside the vacuum chamber 21. Due to the suction pressure, the paracentral region 23 of the eye bearing element 2 is forced towards the outer surface 4 of the eye 5, whereby the contact between the eye bearing element 2 and the outer surface 4 of the eye 5 may be significantly improved.

Furthermore, the guiding device 6 comprises a displaceable shut-off member 25 for selectively shutting off the passageway 8. The shut-off member 25 is displaceable between an open position outside the passageway 8 and a closed position shutting-off the passageway 8. In the shown embodiment, the shut-off member 25 is displaceable in a direction 26 perpendicular to the longitudinal axis of the passageway 8. Preferably, the shut-off member 25 is operably connected to a spring member (not shown) for biasing the shut-off member 25 towards the closed position to close the passageway 8 when the ophthalmic instrument is withdrawn.

In the shown embodiments, the eye bearing element 2 is separate from the guiding device 6. Preferably, the eye bearing element 2 is detachably mounted on the guiding device 6 such that the eye bearing element 2 may be replaced after use.

Fig. 2a to Fig. 2f illustrate different steps of the injection procedure performed with the ophthalmic apparatus 1.

According to a first step (Fig. 2a), the shut-off member 25 of the guiding device 6 is withdrawn towards the open position to allow for the insertion of the injection needle 7' into the passageway 8. The shut-off member 25 may be operated manually.

According to a second step (Fig. 2b), the injection needle 7' is passed through the passageway 8 of the guiding device 6. The first front end 10 of the guiding device 6 serves as a bearing surface for the lower side of the syringe 11 carrying the injection needle 7'. Thus, the bearing surface 10 of the guiding device 6 limits the advancement of the injection needle 7' through the guiding device 6 such that a tip 27 of the injection needle 7' protrudes outside the second front end 12 of the guiding device 6 with a given length. The syringe 11 is filled with the substance 28 for administration into the eye. As an example, substance 28 may be anti-VEGF (Vascular endothelial growth factor) for treating age-related macular degeneration, macula edema due to diabetic retinopathy or retinal central/branch vein occlusion(s) and other diseases leading to macula edema.

According to a third step (Fig. 2c), apparatus 1 is attached to the eye 5, wherein the injection needle 7' penetrates into the eye 5 until the tip 27 of the injection needle 7' reaches the vitreous body of the eye 5. The penetration depth of the injection needle 7' is limited by the eye bearing element 2 coming into abutment against the outer surface 4 of the eye 5. Moreover, the vacuum system is activated to create a negative pressure in the vacuum chamber 21, whereby the eye bearing element 2 is lightly pressed against the outer surface 4 of the eye 5. Then, the substance 28 stored in the syringe 11 is administered to the vitreous body of the eye 5. As an alternative to the vacuum system, apparatus 1 may comprise a spring element arranged for application of a predetermined pressure onto the paracentral region of eye bearing element 2 when the injection needle 7' is introduced into the eye 5 (not shown).

As a variant to steps two and three, the apparatus 1 is positioned on the eye 5, before the injection needle 7' is inserted through the insertion area 13 into the eye 5 (not shown).

According to a fourth step (Fig. 2d), the pressurizing means 17 are activated to further decrease leakage from the orifice created by the injection needle.

According to a fifth step (Fig. 2e), after injection of the substance into the eye 5 the syringe 11 holding the injection needle 7' is retracted from the apparatus 1, whereas the apparatus 1 is maintained in abutment against the eye 5.

According to a sixth step (Fig. 2f), the shut-off member 25 is moved, preferably automatically by means of a spring member, into the closed position, when the injection needle is retracted beyond the shut-off member 25. The shut-off member 25 arranged in the closed position sealingly closes the passageway 8 to maintain the desired pressure inside the pressure chamber 18. At this stage, the apparatus 1 may also be laterally moved (cf. arrows 29) to shift the superficial layers of the eye 5 (conjunctiva and sclera) relative to each other, which may obstruct the orifice created by the injection needle 7' to further prevent leakage therefrom. As a final step, the pressurizing means 17 and the vacuum system are deactivated and the apparatus 1 is removed from the eye 5.

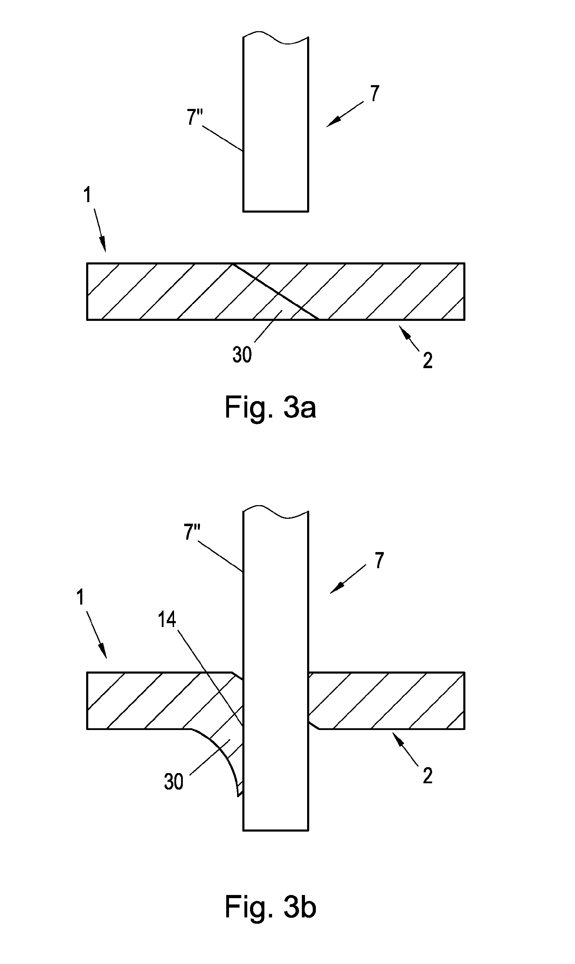

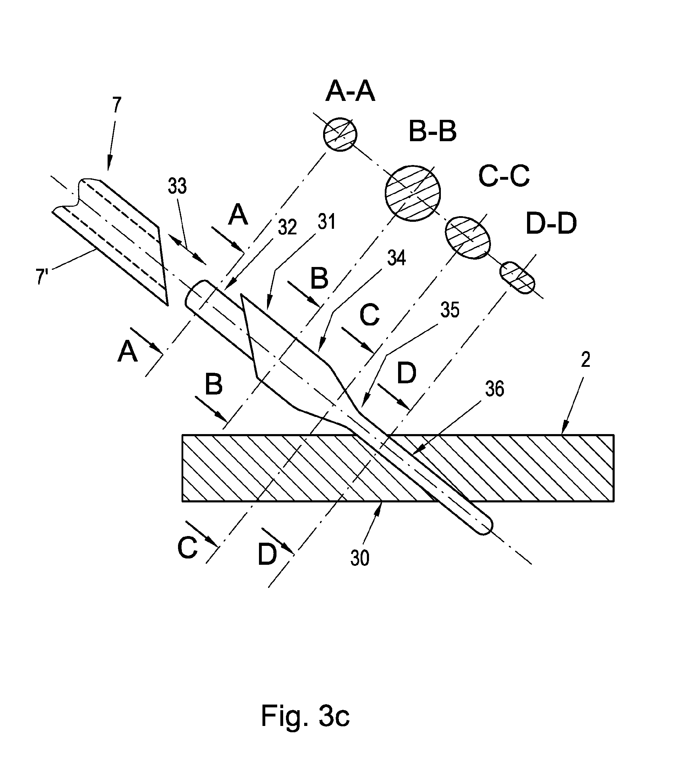

Fig. 3 illustrates a further preferred embodiment for preventing leakage from the eye. In this case, the eye bearing element 2 comprises a flap 30, which may be moved by means of the ophthalmic instrument 7 from a closed position (Fig. 3a), wherein the insertion aperture 14 is closed, and an open position, wherein the insertion aperture 14 is open. Due to the elasticity of the resilient material the flap 30 is transferred back into the closed position when the ophthalmic instrument 7 is withdrawn from the eye bearing element 2 to prevent leakage of eye liquid. This embodiment is particularly advantageous for use with a surgical instrument 7'', as such surgical instruments 7" typically lack a pointed tip suitable for piercing the eye bearing element 2. Thus, flap 30 is pivoted from the closed position into the open position upon insertion of the surgical instrument 7" and returns into the closed position upon retraction of the surgical instrument 7".

On the other hand, Fig. 3c shows a variant of the embodiment according to Fig. 3a, 3b, wherein an injection needle 7' with a pointed tip may be used as ophthalmic instrument 7 for insertion through flap 30. For this purpose, injection needle 7' is provided with an attachment 31 fixable or fixed to the lower end thereof. In an attached state, attachment 31 prolongs injection needle 7' for reliably moving flap 30 between the closed and the open position without risk of damaging eye bearing element 2 due to the pointed tip of injection needle 7'. In the shown embodiment, attachment 31 comprises a connecting portion 32 for detachably connecting attachment 31 with the lower end of injection needle 7'. Here, connecting portion 32 may be snapped on injection needle 7', cf. arrows 33. Thus, in the attached state (not shown), connecting portion 32 of attachment 31 is housed within injection needle 7'. Furthermore, attachment 31 comprises a middle portion 34 which, in the attached state, comes into abutment with the lower end of injection needle 7'. Preferably, middle portion 34 laterally protrudes the injection needle 7' in order to protect the pointed tip of injection needle 7'. Further, attachment 31 comprises a transition portion 35 adjoining middle portion 34, which transition portion 35 is adjoined by an insertion portion 36 at the free end of attachment 31. As can be seen in Fig. 3c, transition portion 35 and insertion portion 36 are flattened, i.e. deviate from a circular cross-section, thereby facilitating opening of flap 30 when attachment 31 is inserted through eye bearing element 2. Attachment 31 further comprises a central channel for guiding the drug from injection needle 7' into the eye 5.

According to the embodiment of Fig. 4, the insertion area 13 of the eye bearing element 2 comprises a permanent aperture 14 for passing the injection needle 7' through the eye bearing element 2. The permanent aperture 14 has a round, preferably circular, cross-sectional shape, which corresponds to the cross-sectional shape of the injection needle 7'. In the shown embodiment, the permanent aperture 14 is arranged in a central region of the eye bearing element 2. The eye bearing element 2 further comprises an annular sealing surface 15 around the permanent aperture 14. When the ophthalmic instrument 7, here an injection needle 7', is passed through the permanent aperture 14, the sealing surface 15 of the eye bearing element 2 sealingly bears on the outer surface of a sealing section of the injection needle 7'. The diameter of the permanent aperture 14 is significantly less than the outer diameter of the injection needle 7' in the sealing section. Thus, the resilient sealing surface 15 of the eye bearing element 2 is pressed against the sealing section of the injection needle 7' when passed through the permanent aperture 14 widened by the insertion of the injection needle 7'. Moreover, the diameter of the permanent aperture 14 is smaller than the diameter of the passageway 8 of the guiding device 6. For ensuring a tight fit between the sealing surface 15 of the eye bearing element 2 and the sealing section of the injection needle 7', the resilient material of the eye bearing element 2 preferably comprises or consists of a natural or synthetic rubber material. When the injection needle 7' is withdrawn from the eye 5 and the eye bearing element 2, the permanent aperture 14, widened by the introduction of the injection needle 7', is elastically reduced to its original size. In this state, the permanent aperture 14 is dimensioned such that leakage of eye liquid therethrough is largely prevented.

In the examples shown in Fig. 1 to Fig. 4, the eye bearing element 2 is made in one piece from the resilient material. However, in an alternative embodiment the injection area 13 and the paracentral region 23 of the eye bearing element 2 are formed by different materials.

## Claims

1. An apparatus (1) for application on an eye (5), comprising an eye bearing element (2) with a lower surface (3) for bearing on the eye (5), the eye bearing element (2) comprising an insertion area (13) for passing an ophthalmic instrument (7), in particular an injection needle (7') or a surgical instrument (7"), therethrough, **characterized in that** the eye bearing element (2), at least in the insertion area (13), is made from a resilient material arranged such that an insertion aperture (14) formed in the insertion area (13) upon insertion of the ophthalmic instrument (7) is at least substantially closed when the ophthalmic instrument (7) is retracted from the eye bearing element (2).

2. An apparatus (1) according to claim 1, **characterized in that** the eye bearing element (2) comprises a continuous insertion area (13) arranged for producing the insertion aperture (14) by means of piercing the ophthalmic instrument (7) therethrough.

3. An apparatus (1) according to claim 1, **characterized in that** the eye bearing element (2) comprises a flap (30) transferrable between a closed position, wherein the insertion aperture (14) is substantially closed, and an open position, wherein the insertion aperture (14) is open, wherein the flap (30) is biased towards the closed position.

4. An apparatus (1) according to claim 1, **characterized in that** the insertion area (13) of the eye bearing element (2) comprises a permanent aperture (14) arranged for sealingly accommodating the ophthalmic instrument (7).

5. An ophthalmic apparatus (1) according to claim 4, **characterized in that** a diameter of the permanent aperture (14) of the injection area (13) is smaller than a standardized diameter of an injection needle (7'), in particular less than 0,36 mm.

6. An apparatus (1) according to any one of claims 1 to 5, **characterized in that** the resilient material of the eye bearing element (2) comprises a natural or synthetic rubber material.

7. An apparatus (1) according to any one of claims 1 to 6, **characterized in that** the eye bearing element (2) is made in one piece from the resilient material.

8. An apparatus (1) according to any one of claims 1 to 7, **characterized in that** the lower surface (3) of the eye bearing element (2) is curved for matingly bearing on the eye (5), the radius of curvature of the eye bearing element (2) preferably being less than the radius of curvature of a human eye (5) around the injection area (13) posterior to the limbus.

9. An apparatus (1) according to any one of claims 1 to 8, **characterized in that** the eye bearing element (2) is connected to a guiding device (6) for guiding the ophthalmic instrument (7) between a retracted position, wherein the tip (27) of the ophthalmic instrument (7) is retracted inside the guiding device (6), and an inserted position, wherein the tip (27) of the ophthalmic instrument (7) protrudes outside the guiding device (6).

10. An apparatus (1) according to claim 9, **characterized in that** the guiding device (6) comprises pressurizing means (17) for passing a pressurized fluid to the insertion area (13) of the eye bearing element (2).

11. An apparatus (1) according to claim 10, **characterized in that** the pressurizing means (17) comprise a pressure chamber (18) inside the guiding device (6), the pressure chamber (18) having an inlet (19') for connecting a source of pressurized fluid thereto and an outlet (20) for passing the pressurized fluid to the insertion area (13) of the eye bearing element (2).

12. An apparatus (1) according to claim 11, **characterized in that** the guiding device (6) comprises a passageway (8) for passing the ophthalmic instrument (7) therethrough, the passageway (8) extending to the pressure chamber (18), wherein the guiding device (6) further comprises a shut-off member (25) for shutting off the passageway (8) when the ophthalmic instrument (7) is arranged in the retracted position.

13. An apparatus (1) according to any one of claims 1 to 12, **characterized in that** the eye bearing element (2) comprises at least one paracentral opening (24) outside the insertion area (13).

14. An apparatus (1) according to claim 13, **characterized in that** the at least one paracentral opening (24) of the eye bearing element (2) is connected to at least one vacuum chamber (21) inside the guiding device (6), the vacuum chamber (21) having an intake (22) for connecting a vacuum source thereto.

15. An apparatus (1) according to any one of claims 1 to 14, **characterized in that** the eye bearing element (2) is made in one piece with the guiding device (6) or the eye bearing element (2) is detachably mounted on the guiding device (6).
